(19) Europäisches Patentamt
European Patent Office
Office européen des brevets

(11) Veröffentlichungsnummer: **0 284 898 A2**

(12) # EUROPÄISCHE PATENTANMELDUNG

(21) Anmeldenummer: 88104201.4

(22) Anmeldetag: 16.03.88

(51) Int. Cl.⁴: **C07K 7/06**, A61K 37/02

(30) Priorität: 02.04.87 EP 87104867

(43) Veröffentlichungstag der Anmeldung: 05.10.88 Patentblatt 88/40

(84) Benannte Vertragsstaaten: BE CH DE FR GB IT LI SE

(71) Anmelder: Max-Planck-Gesellschaft zur Förderung der Wissenschaften e.V.
Bunsenstrasse 10
D-3400 Göttingen(DE)

(72) Erfinder: Wünsch, Erich, Prof. Dr.
Midgardstrasse 16
D-8132 Tutzing(DE)
Erfinder: Moroder, Luis, Prof.Dr.
Lena-Christ-Strasse 12
D-8033 Martinsried(DE)

(74) Vertreter: Lederer, Franz, Dr. et al
Van der Werth, Lederer & Riederer
Patentanwälte Lucile-Grahn-Strasse 22
D-8000 München 80(DE)

(54) **Hinge-Peptid, Verfahren zu seiner Herstellung und seine Verwendung zur Herstellung synthetischer Immunogene.**

(57) Die Erfindung betrifft ein Hinge-Peptid derallgemeinen Formel I, welches zur Herstellung synthetischer Immunogene verwendet werden kann. Die Erfindung umfaßt auch ein Verfahren zur Herstellung dieses Hinge-Peptids und ein Verfahren zur Herstellung synthetischer Immunogene, wobei man das Hinge-Peptid als Trägerprotein einsetzt.

Hinge - Peptid

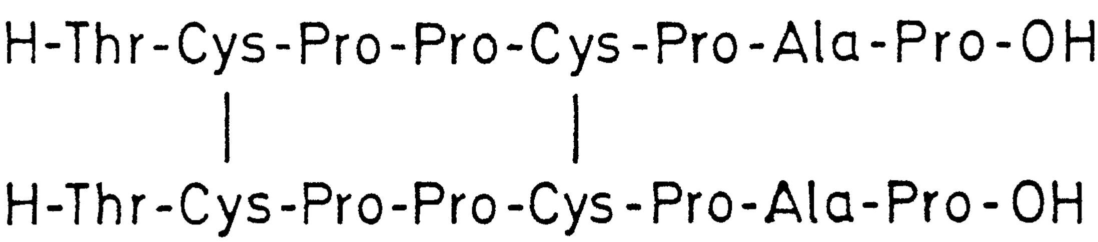

(I)

EP 0 284 898 A2

# Hinge-Peptid, Verfahren zu seiner Herstellung und seine Verwendung zur Herstellung synthetischer Immunogene

Untersuchungen der Arbeitsgruppe Sela ((1) Arnon, R., and Sela, M. (1969) Proc. Natl. Acad. Sci. USA 62, USA 62, 163-170; (2) Arnon, R., Maron, E., Sela, M., and Anfinsen, G.B. (1971) Proc. Natl. Acad. Sci. USA 68, 1450-1454) am Protein Lysozymin mittels synthetischer Partialsequenzen haben die Basis geschaffen für spätere Studien über den möglichen Einsatz von Partialsequenzen aus Virus-Hüllproteinen oder Bakterientoxinen, die antigenen Regionen dieser Pathogene entsprechen, zur Erstellung nicht infektiöser Ersatzvakzine ((3) Sela, M. (1974) Bull. Inst. Pasteur 72, 73-86; (4) Lerner, R.A. (1982) Nature (London) 199, 592; (5) Sela, M. (1983) Biopolymers 22, 415-424). Solche synthetische Vakzine sollten das immunogene Potential natürlicher Pathogene in bezug auf dauerhafte Immunantwort auf T-und B-Zellen-Ebene nachahmen und die $T_h$-und B-Zellen induzierenden Determinanten voll exprimieren.

Zur Zeit basiert das allgemeine Verfahren dieser Versuche auf Verknüpfung synthetischer Peptidsequenzen antigener Sequenzbereiche von Proteinen mit Trägerproteinen oder polymeren Verbindungen vielfach stark immunogener Eigenschaften mittels Vernetzungsreagentien. Spezifische Verknüpfungsreagentien finden diesbezüglich verstärkte Aufmerksamkeit ((7) Stevens, V.C. (1986) in Synthetic Peptides as Antigens, CIBA Foundation Symposium 119, Wiley, Chichester, pp. 200-225), wobei eigene Untersuchungen der Erfinder in diesem Zusammenhang zu erheblichen Fortschritten geführt haben ((8) Geiger, R., Moroder, L., and Wünsch, E. (1984) in Peptides 1984 (Ragnarsson, U., Ed.) Almquist und Wiksell Int., Stockholm, pp. 451-456). Solche Peptid/Proteinkonjugate sind bereits für Immunisierungen an Versuchstieren eingesetzt worden. Die Immunantwort kann durch Zusatz oder Einbau bekannter Immunadjuvantien noch gesteigert werden.

Diese Untersuchungen haben zu den Erkenntnissen geführt ((9) Review: Synthetic Peptides as Antigens, CIBA Foundation Symposium 119, Wiley, Chichester, 1986), daß 1.) dominierende Immunepitope von Virus-Hüllproteinen und Bakterien-Toxinen Definierten Proteinsequenzen in räumlich fixierter Form entsprechen, und daß 2.) Träger solcher Epitopsequenzen nicht nur einer Depotwirkung dienen, sondern auch eine entscheidende Rolle in der Präsentation der Epitopsequenzen für eine spezifische Erkennung seitens der $T_h$-und B-Zellen spielen.

Aus der DE-Al-34 30 894 ist ein synthetischer Impfstoff gegen Cholera und Hitze-labiles E. coli-Toxin bekannt, der ein Verknüpfungsprodukt aus einem hochmolekularen Träger mit einem Polypeptid umfaßt, das einem Teil der Sequenz der Untereinheit B des natürlichen Choleratoxins entspricht. Der Träger kann ein geeignetes Toxoid sein oder ein synthetisches Polymer mit hohem Molekulargewicht, wie z.B. ein Alanin-Lysin-Polymer mit einem Molekulargewicht von mindestens etwa 50.000.

Es ist bekannt, daß die Peptidketten der Immunglobuline über eine "Hinge"-Region miteinander verbunden sind, so daß beispielsweise die Y-förmige Struktur des IgG entstehen kann. Die Y-Form ermöglicht die bei der Antigenbindung notwendige Flexibilität des IgG-Moleküls.

Aufgabe der Erfindung ist die Schaffung eines vielseitig anwendbaren Zentralmoleküls, das als "Hinge"-Region für die Verknüpfung von Peptidsequenzen antigener Sequenzbereiche von Proteinen brauchbar ist.

Den Anforderungen eines effizienten Immunogens in bezug auf reproduzierbare Immunantwort mit letztlich neutralisierenden Eigenschaften kann sicherlich nur durch Synthese eines Moleküls definerter Struktur unter Einbau eines oder mehrerer Epitope von Pathogenen erfolgreich entsprochen werden. Für den Aufbau solcher synthetischer Miniproteine als Immunogene ist ein Zentralmolekül definierter Struktur, versehen mit mehreren unterschiedlichen Verankerungsstellen, als Trägergerüst notwendig. Ein solches Skelettmolekül haben die Erfinder nun der Primärstruktur der Immunoglobuline in Form des Hinge-Peptids entnommen. Es handelt sich dabei um ein doppelstrangiges heterodetes zyklisches Cystin-Peptid der Formel I

```
H-Thr-Cys-Pro-Pro-Cys-Pro-Ala-Pro-OH
      |           |
      |           |                        (I)
H-Thr-Cys-Pro-Pro-Cys-Pro-Ala-Pro-OH
```

Alle Aminosäuren in dieser Formel besitzen die L-Konfiguration. Die vier Verankerungsstellen, die sich durch Anbau von drittfunktionellen Aminosäureresten beliebig am N-und C-Terminus erweitern lassen, können selektiv geschützt werden. Dadurch wird der gezielte Anbau mehrerer Eptiopse quenzen gleicher oder unterschiedlicher Primärstruktur in linearer oder zyklischer Form möglich. Zusätzlich ist eine covalente Anknüpfung von Adjuvantien bekannter Natur, z.B. von Muramylpeptiden oder Fettsäurederivaten von Aminosäuren etc., jederzeit möglich.

Es ist möglich an dem Zentralmolekül der Formel I gewisse Änderungen vorzunehmen ohne die Eigenschaften dieses Peptids zu beeinträchtigen. So ist es beispielsweise möglich, den Aminosäurerest Pro an einer oder mehreren Positionen durch einen Aminosäurerest, der die gleiche räumliche Anordnung gewährleistet, zu ersetzen, so daß das erfindungsgemäße Hinge-Peptid mit der allgemeinen Formel Ia

```
H-Thr-Cys-X-X-Cys-X-Ala-X-OH
      |       |                      (Ia)
H-Thr-Cys-X-X-Cys-X-Ala-X-OH
```

bezeichnet werden kann, worin X Pro oder der Rest einer dem Prolin in sterischer Hinsicht äquivalenten Aminosäure ist.

Auch andere Substitutionen sind denkbar.

Grundsätzlich ist die Doppelkette der Hinge-Region in "paralleler" und "antiparalleler" Anordnung möglich. Die parallele Anordnung ist stabiler und wird daher bevorzugt.

Das erfindungsgemäße Hinge-Peptid besitzt den Vorteil, daß es relativ klein und starr ist. Die daraus resultierende Stabilität überträgt sich auch auf die aus dem Hinge-Peptid aufgebauten Immunogene, was beispielsweise eine Enzymstabilität vorteilhaft beeinflußt.

Das erfindungsgemäße Hinge-Peptid besitzt mehrere Funktionen, die teilweise oder vollständig in an sich bekannter Weise durch übliche Schutzgruppen, beispielsweise BOC, FMOC (Fluorenylmethoxycarbonyl), Nps (2-Nitrophenylsulfenyl), tBu (= tert. Butylester und tert. Butylether), Acm (Acetylaminomethyl), Trityl geschützt werden können.

Theoretisch wäre es möglich, das erfindungsgemäße Hinge-Peptid durch Abspaltung aus natürlichen Immunoglobulinen zu gewinnen, dies ist aber nicht nur wegen des unverhältnismäßig großen Aufwands unpraktikabel, sondern auch wegen der Gefahr des Einschleppens von Verunreinigungen, die ähnlich wäre der Gefahr bei der Herstellung von Vakzinen aus natürlichen Quellen, die gerade durch die Herstellung synthetischer Vakzine ausgeschaltet werden soll.

Es bieten sich verschiedene Wege für die Synthese des erfindungsgemäßen Hinge-Peptids an.

Weg A (Formelschema A)

Unter Anwendung bekannter Methoden der konventionellen Peptidsynthese in Lösung ((10) Houben-Weyl, Methoden der Organischen Chemie, Vol. 15 I/II (Wünsch E., ed.) Georg Thieme, Stuttgart, 1984) werden die Peptid-Derivate II und III der beiden Ketten des Hinge-Peptids erstellt. Nach anschließender reduktiver Abspaltung der tert.-Butylthio-Reste von II und III mittels Phosphinen ((11) Moroder, L., Gemeiner M., Göhring, W., Jaeger, E., Thamm, P., and Wünsch, E. (1981) Biopolymers 20, 17-37) werden die Cystein-Peptide mit Azodicarbonsäure-Derivaten ((12) Wünsch E., Romani S., Hoppe-Seyler's Z. Physiol. Chem. 363 (1982) 449-453) selektiv zum Mono-Cystin-Peptid IV verknüpft. Die zweite Cystin-Brücke wird nunmehr durch Jodoxidation des Peptid-Derivats IV nach aus der Literatur bekannter Methode ((13) Kamber B., and Rittel W. (1968) Helv. Chim. Acta 51, 2061-2064) selektiv erstellt. Dadurch werden Peptid-Derivate der allgemeinen Formel V zugänglich.

Weg B (Formelschema B)

Alternativ zu Weg A wird das Oktapeptid-Derivat VI nach Verfahren der Konventionellen Peptidsynthese erstellt. Abspaltung der beiden tert.-Butylthiogruppen erfolgt wiederum durch Reduktion mit Phosphinen (-(11) Moroder, L., Gemeiner, M., Göhring, W., Jaeger, E., Thamm, P. and Wünsch, E. (1981) Biopolymers 20, 17-31). Das resultierende Bis-Cystein-Peptid-Derivat VII wird durch Luftoxidation bei Kon zentrationen von $10^{-3}$ -$10^{-4}$ M in das Hinge-Peptid-Derivat VIII überführt. Die parallele Anordnung der beiden Ketten ist energetisch so bevorzugt, daß ausschließlich das Produkt der Formel VIII entsteht. Dies wurde durch Vergleichsuntersuchungen von Hinge-Peptid-Präparationen aus Weg A (gezielte Disulfidbrückenbildung) und Weg B bewiesen.

Die Strukturen der Hinge-Peptide können mittels verschiedenster analytischer Methoden - z.B. Molekulargewichtsbestimmung durch osmometrische Methoden, Aminosäureanalysen nach saurer Hydrolyse und enzymatischem Abbau, Razemattest und hochauflösende Kernresonanzspektroskopie - bestätigt werden. Die Einheitlichkeit dieser Produkte wurde mittels chromatographischer Analysetests bewiesen.

Das erfindungsgemäße Hinge-Peptid kann in vorteilhafter Weise zur Herstellung synthetischer Immunogene verwendet werden.

Die Erfindung umfaßt daher auch ein Verfahren zur Herstellung synthetischer Immunogene, wobei man ein erfindungsgemäßes Hinge-Peptid als Trägerprotein einsetzt und dieses in an sich bekannter Weise mit Peptidsequenzen, vorzugsweise synthetischen Peptidsequenzen, antigener Sequenzbereiche von Proteinen verknüpft.

Eine solche Synthese von Epitop-Konjugaten wird am Beispiel des Gastrin/Hinge-Peptids als Modell nachstehend erläutert.

Weg A (Formelschema C)

Das $N^{\alpha}$-und seitenkettengeschützte Gastrin-Peptid der Sequenz 6-17 des Norleucine-15-Analogons von Human-Little-Gastrin-I IX wurde mittels der Misch-Anhydrid-Methode an beide N-Termini des Hinge-Peptid-Derivats V mit bester Ausbeute angeknüpft. Nach Schutzgruppenabspaltung von X mit HCl in Anwesenheit von 2-Methylindol und Trifluoressigsäu re/2-Methylindol, konnte das gewünschte Gastrin-Hinge-Peptid-Konjugat XI durch Gelfiltration und Verteilungschromatographie an Cellulose als einheitliches Produkt gewonnen werden.

Weg B (Formelschema D)

Wiederum wurde das geeignet geschützte Gastrin-Peptid IX mit dem Oktapeptid VI mittels der Misch-Anhydrid-Methode zu XII verknüpft. Nach zweistufiger Abspaltung der säurelabilen Schutzgruppen und säulenchromatographischer Zwischenreinigung wurden nunmehr von resultierenden Peptidderivat XIII die Cystein-Schutzgruppen reduktiv nach dem Phosphin-Verfahren entfernt. Das erhaltene Bis-Cystein-Peptid wurde durch Luftoxidation in o.g. Konzentrationen in besten Ausbeuten in das Gastrin-Hinge-Peptid-Konjugat XI überführt.

Präparate aus Weg A und Weg B wurden in allen durchgeführten analytischen Bestimmungen als identisch gefunden.

Immunisierungen mit Gastrin-Hinge-Peptid-Konjugat

Immunisierungen an Kaninchen wurden mit diesem Modell-Konjugat unter Zugabe von Freundschem Adjuvans nach bekannten Verfahren ((14) Turkelson, C.M. Dale, W.E., Reidelberger, R., und Solomon, T.E. (1986) Reg. Peptides 15, 205-217) durchgeführt. Die ermittelten Antigastrin-Antisera-Titer waren vergleichbar mit den Antikörper-Titern, die unter Anwendung koventioneller Gastrin-Konjugate, z.B. Gastrin/Ribonuclease oder Gastrin/Rinderserum Albumin, gewonnen wurden ((8) Geiger, R., Moroder, L., und Wünsch, E. (1984) in Peptides 1984 (Ragnarsson, U., ed.) Almquist and Wiksell Int., Stockholm, pp. 451-456).

Beispiel 1a

Nps-Thr(tBu)-Cys(Acm)-Pro-Pro-Cys(StBu)-Pro-Ala-Pro-OH (IIb)

Schmp. 100-105°C; $[\alpha]_D^{20}$ = - 205,3° bzw. $[\alpha]_{546}^{20}$ = - 244,9° (c = 1, Methanol); Aminosäure-Analyse: Thr 0,78 (1) berechnete Werte in Klammern Pro 3,93 (4) Ala 1,00 (1) Cys 1,99 (2); Razemattest: D-Ala 0,4 %; D-allo-Thr 1 %; D-Pro 1 %.

Elementaranalyse für $C_{50}$ $H_{76}$ $N_{10}$ $O_{13}$ $S_4$ .$H_2O$ (1171,5)

Ber.:

C 51,26 H 6,71 N 11,96 S 10,95

Gef.:

C 51,48 H 6,72 N 11,59 S 10, 76

Beispiel 1b

H-Thr(t-Bu)-Cys(Acm)-Pro-Pro-Cys-Pro-Ala-Pro-OH (IIIb)

Durch Reduktion von IIb mit 5 Äquiv. Tributylphosphin in 95 %igem Trifluoräthanol (3 h bei Raumtemperatur) in 93 % Ausbeute gewonnen.
$[\alpha]_D^{20}$ = - 18,2° bzw. $[\alpha]_{546}^{20}$ = - 217,3° (c = 1, Methanol)
Elementaranalyse für $C_{40}H_{65}N_9O_{11}S_2$ (912,15)
Ber.:
C 52,67 H 7,18 N 13,82 S 7,03
Gef.:
C 52,63 H 7,20 N 13,49 S 7,09

Beispiel 1c

[H-Thr(tBu)-Cys(Acm)-Pro-Pro-Cys-Pro-Ala-pro-OH]$_2$ (IVb)

Oxidation von IIIb mit 0,5 Äquiv. Azodicarbonsäure-di-tert.-butylester in Dimethylformamid;
Ausbeute 81 %;Schmp. 180-185°C (Zers.); $[\alpha]_D^{20}$ = - 204,0° bzw. $[\alpha]_{546}^{20}$ = - 243,4° (c = 1, 80 %ige Essigsäure).
Aminosäure-Analyse:
Thr 1,34 (2) Pro 7,92 (8) Ala 2,00 (2) Cys 4,03 (4); Peptidgehalt: 92 %

Beispiel Id

[H-Thr(tBu)-Cys-Pro-Cys-Pro-Ala-Pro-OH]$_2$ (Vb)

Langsames Zutropfen von IVb in 80 %iger Essigsäure (c = 2 x $10^{-3}$ M) zu Jod in 80 %iger Essigsäure (5 Äquiv., c = 1 x $10^{-2}$ M); Endkonzentration an Peptid 1 x $10^{-3}$ M.
Ausbeute: 74 %; Schmp. 215-220°C (Zers.); $[\alpha]_D^{20}$ = - 321,0° bzw. $[\alpha]_{546}^{20}$ = -382,6° (c = 1; 80 %ige Essigsäure).
Aminosäure-Analyse:
Thr 1,60 (2) Pro 7,83 (8) Ala 2,00 (2) (2) Cys 4,00 (4); Peptidgehalt: 98 %
Razemattest:
D-Ala 0,8 %; D-allo-Thr 0,8 %; D-Pro < 1%.
Osmometrische Molgewichtsbestimmung:
gef. $M_r$ = 1.470
theor. $M_r$ = 1.678,1
Elementaranalyse für $C_{74}H_{116}N_{16}O_{20}S_4 . 5H_2O$ (1.768,2)
Ber.:
C 50,27 H 7,18 N 12,67 S 7,25
Gef.:
C 50,20 H 7,08 N 12,70 S 7,02

Beispiel 1e

[H-Leu-[Glu(OtBu)]$_5$-Ala-Tyr(tBu)-Gly-Trp-Nle-Asp(OtBu)-Phe-Thr(tBu)-Cys-Pro-Pro-Cys-Pro-Ala-Pro-OH]$_2$ (Xb)

Umsetzung von Vb (1 Äquiv.) mit 3 Äquiv. Nps-Leu-[Glu(OtBu)]$_5$-Ala-Tyr(tBu(-Gly-Trp-Nle-Asp(OtBu)-Phe-OH (IX) mittels der Misch-Anhydrid-Methode und anschließende NpsAbspaltung mit HCl in N-Methylpyrrolidon in Anwesenheit von 2-Methylindol. Reinigung durch Gelfiltration an LH60 (N-Methylpyrrolidon als Lösungsmittel); Ausbeute: 35 %.

Aminosäureanalyse: Asp 2,14 (2) Thr 176 (2) Glu 9,40 (10) Pro 8,22 (8) Gly 2,12 (2) Ala 4,20 (4) Cys 4,22 (4) Leu 1,86 (2) Nle/Tyr 4,16 (4) Phe 2,18 (2); Peptidgehalt: 85 % ($M_r$ = 5.759,9 als Dihydrochlorid).

Beispiel 1f

[H-Leu-[Glu]$_5$-Ala-Tyr-Gly-Trp-Nle-Asp-Phe-Thr-Cys-Pro-Pro-Cys-pro-Ala-Pro-OH]$_2$ (XIb)

Verbindung Xb wird mit 99 % Trifluoressigsäure/Anisol (10:1) in Anwesenheit von 2-Methylindol (50 Äquiv.) behandelt. Das Rohprodukt wird durch Gelfiltration an Fractogel HW-40S (Eluens: 1 M Ammoniumacetat/2-Butanol/2-Propanol, 100:16:1,5, pH 7,2) und durch Verteilungschromatographie an Cellulose (Eluens: 0,05 M Ammoniumacetat/2-Butanol/1-Butanol/2-Propanol, 20:10:4:4, pH 5,5) gereinigt.

Aminosäure-Analyse des HCl-Hydrolysats:

Asp 2,09 (2) Thr 1,72 (2) Glu 9,59 (9) Pro 7,93 (8) Gly 2,14 (2) Ala 3,99 (4) Cys 4,24(4) Leu 2,00 (2) Nle/Tyr 3,95 (4) Phe 2,03 (2) Trp 1,92 (2); Peptidgehalt: 86,6 % ($M_r$ = 4.789,4);

XIb wurde nach Reduktion mit Dithiothreitol, S-Carboxymethylierung mit Jodessigsäure zur Aminosäureanalyse mit Aminopeptidase M/Prolidase verdaut.

Aminosäureanalyse: Asp 2,06 (2) Thr 1,84 (2) Glu 9,96 (10) Pro* 6,60 (8) Gly 2,00 (2) Ala 3,94 (4) Nle/Tyr 3,82 (4) Phe 2,02 (2) Trp 1,80 (2) Leu (wegen TRIS-Puffer überlappung) und S-Carboxymethyl-Cystein nicht bestimmt.

*) Auch Prolidase verdaut nicht quantitativ Pro-Pro-Sequenzen

## Ansprüche

1.) Hinge-Peptid der allgemeinen Formel

```
H-Thr-Cys-X-X-Cys-X-Ala-X-OH
       |       |                    (Ia)
H-Thr-Cys-X-X-Cys-X-Ala-X-OH
```

worin die mit X bezeichneten Aminosäurereste unabhängig voneinander Pro oder der Rest einer dem Prolin in sterischer Hinsicht äquivalenten Aminosäure darstellen.

2.) Hinge-Peptid nach Anspruch 1 der Formel

```
H-Thr-Cys-Pro-Pro-Cys-Pro-Ala-Pro-OH
       |           |                    (I)
H-Thr-Cys-Pro-Pro-Cys-Pro-Ala-Pro-OH
```

3.) Hinge-Peptid nach Anspruch 1 oder 2 in voll-oder teilgeschützter Form.

4.) Verfahren zur Herstellung eines Hinge-Peptids nach einem der Ansprüche 1-3, dadurch gekennzeichnet, daß man

a) unter Anwendung bekannter Methoden der konventionellen Peptidsynthese die Peptid-Derivate

$R_1$-Thr($R_2$)-Cys($Z_1$)-X-X-Cys($Z_2$)-X-Ala-X-$OR_3$ (IIa)

und

$R_4$-Thr($R_2$)-Cys($Z_1$)-X-X-Cys($Z_2$)-X-Ala-X-$OR_5$ (IIIa)

worin $R_1$-$R_5$ H oder in der Peptidchemie übliche Schutzgruppen, vorzugsweise

$R_1$ = H; BOC; FMOC; Nps;
$R_2$ = H; tBu;
$R_3$ = H; tBu;
$R_4$ = H; BOC; FMOC; Nps;
$R_5$ = H; tBu

und von $Z_1$ und $Z_2$ das eine Acm oder Trityl und das andere StBu bedeuten,
der beiden Ketten des Hinge-Peptids erstellt, alsdann die tert.-Butylthio-Schutzgruppen des Cysteinrestes mit Phosphinen, beispielsweise $(C_4H_9)_3P$, reduktiv abspaltet und die freigewordenen Cysteinreste der Ketten IIa und IIIa mit einem Azodicarbonsäure-Derivat, beispielsweise BOC-N = N-BOC, selektiv zum Mono-Cystin-Peptid

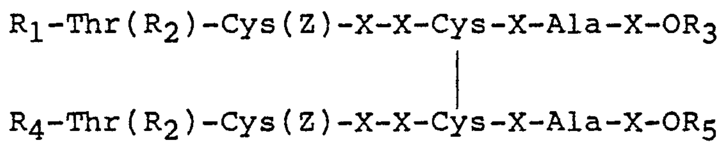

(IVa)

verknüpft und dieses durch Jodoxidation in an sich bekannter Weise zu

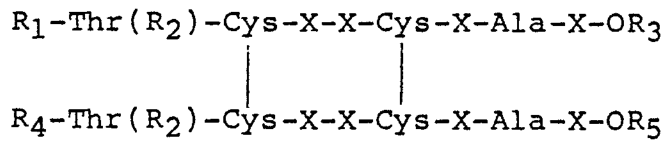

(Va)

oxidiert, worauf dann die Schutzgruppen $R_1$-$R_5$ in an sich bekannter Weise gegebenenfalls selektiv abgespalten werden,
oder

b) unter Anwendung bekannter Methoden der konventionellen Peptidsynthese das Oktapeptid-Derivat
$R_1$-Thr($R_2$)-Cys(StBu)-X-X-Cys(StBu)-X-Ala-X-$OR_3$ (VIa)
worin $R_1$ = H; BOC; FMOC; Nps;
$R_2$ = H; tBu;
$R_3$ = H; tBu;
bedeuten,
erstellt, alsdann die tert.-Butylthio-Schutzgruppen der Cysteinreste mit Phosphinen, beispielsweise $(C_4H_9)_3P$, reduktiv abspaltet und das resultierende Bis-Cystein-Peptid-Derivat
$R_1$-Thr($R_2$)-CysX-X-CysAla-X-$OR_3$ (VIIa)
durch Luftoxidation in das Hinge-Peptid-Derivat

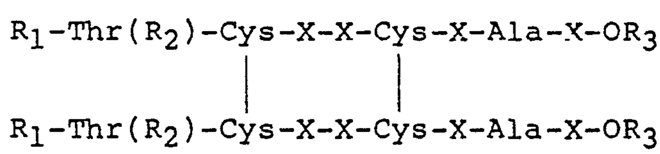

(VIIIa)

überführt,
aus dem die Schutzgruppen $R_1$-$R_3$ in an sich bekannter Weise gegebenenfalls selektiv abgespalten werden.

5.) Verwendung eines der Hinge-Peptide nach einem der Ansprüche 1-3 zur Herstellung synthetischer Immunogene.

6.) Verwendung eines der Hinge-Peptide nach einem der Ansprüche 1-3 zur Synthese eines Gastrin/Hinge-Peptid Konjugats.

7.) Verfahren zur Herstellung synthetischer Immunogene, dadurch gekennzeichnet, daß man ein Hinge-Peptid nach einem der Ansprüche 1-3 als Trägerprotein in an sich bekannter Weise mit Peptidsequenzen antigener Sequenzbereiche von Proteinen und gegebenenfalls mit Immunadjuvantien verknüpft.

16. März 1988

# Hinge-Peptid

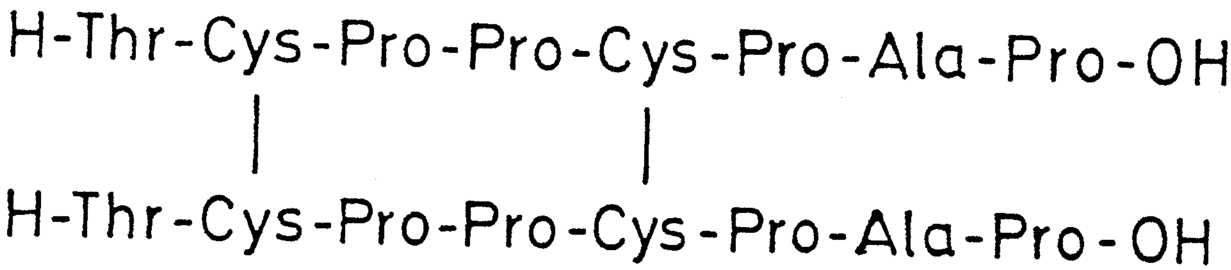

(I)

# FORMELSCHEMA A

Synthese Hinge-Peptid-Derivate

Weg A:

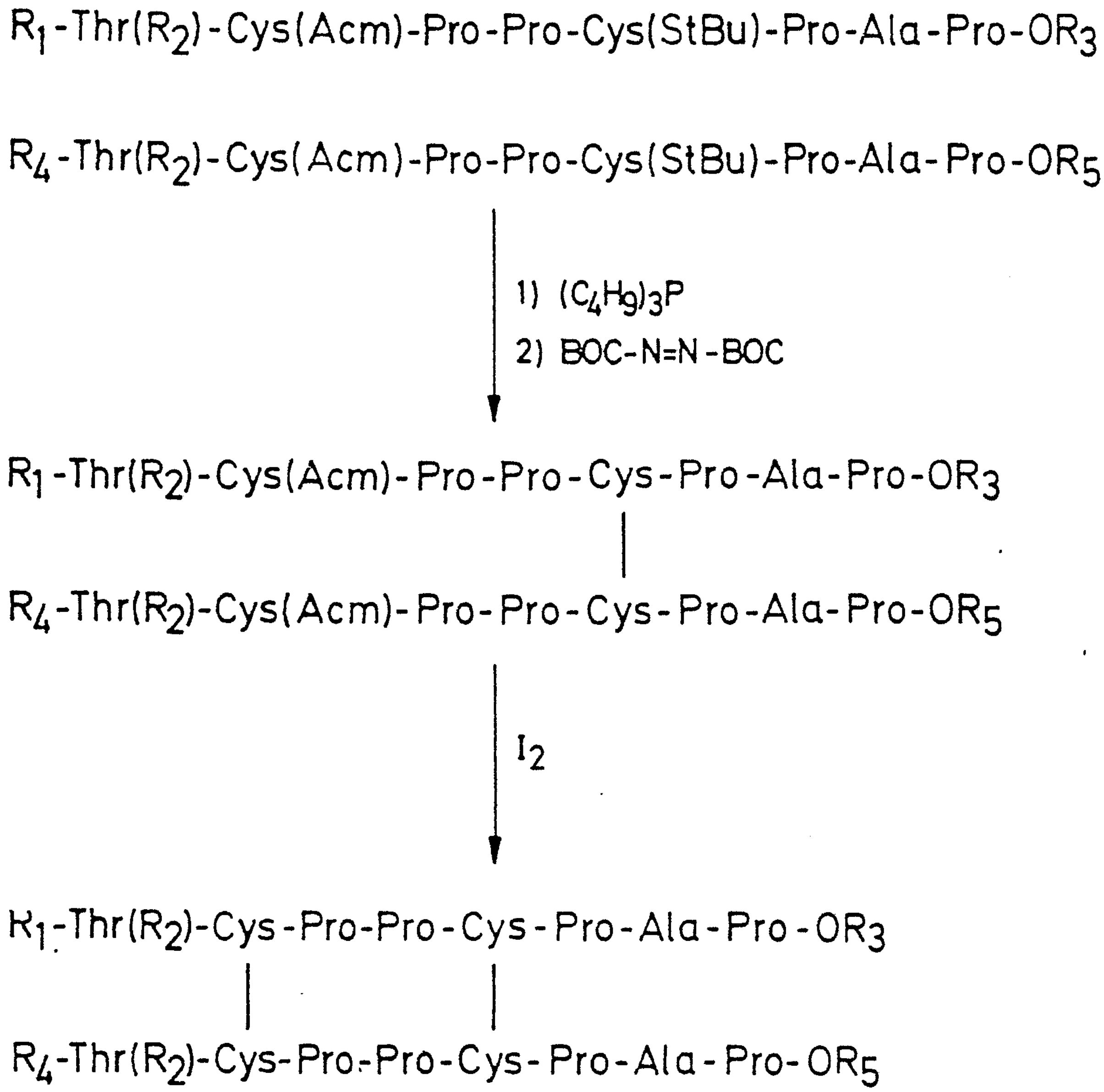


(II)

(III)

(IV)

(V)

# FORMELSCHEMA B

Synthese Hinge-Peptid-Derivate

Weg B:

$R_1$-Thr($R_2$)-Cys(StBu)-Pro-Pro-Cys(StBu)-Pro-Ala-Pro-$OR_3$ (VI)

$(C_4H_9)_3P$

$R_1$-Thr($R_2$)-Cys-Pro-Pro-Cys-Pro-Ala-Pro-$OR_3$ (VII)

$O_2$

$R_1$-Thr($R_2$)-Cys-Pro-Pro-Cys-Pro-Ala-Pro-$OR_3$

$R_1$-Thr($R_2$)-Cys-Pro-Pro-Cys-Pro-Ala-Pro-$OR_3$ (VIII)

# FORMELSCHEMA C

Synthese des Gastrin/Hinge-Peptid Konjugats

Weg A:

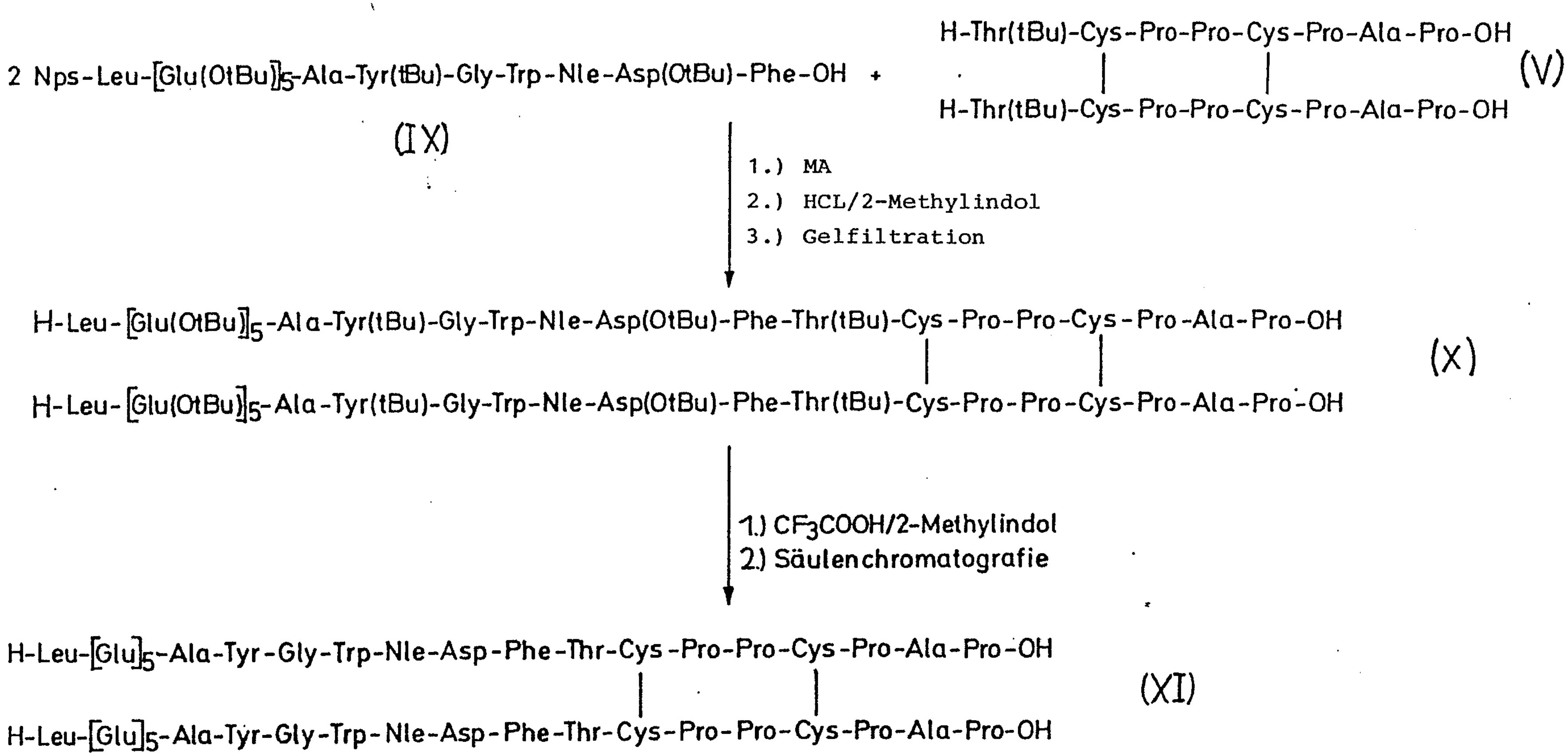


(IX) (V) (X) (XI)

# FORMELSCHEMA D

Synthese des Gastrin/Hinge-Peptid Konjugats

Weg B :

Nps-Leu-[Glu(OtBu)]$_5$-Ala-Tyr(tBu)-Gly-Trp-Nle-Asp(OtBu)-Phe-OH + H-Thr(tBu)-Cys(StBu)-Pro-Pro-Cys(StBu)-Pro-Ala-Pro-OH

(IX) (VI)

M.A.

Nps-Leu-[Glu(OtBu)]$_5$-Ala-Tyr(tBu)-Gly-Trp-Nle-Asp(OtBu)-Phe-Thr(tBu)-Cys(StBu)-Pro-Pro-Cys(StBu)-Pro-Ala-Pro-OH (XII)

1) HCl / 2-Methylindol
2) $CF_3COOH$ / 2-Methylindol

H-Leu-[Glu]$_5$-Ala-Tyr-Gly-Trp-Nle-Asp-Phe-Thr-Cys(StBu)-Pro-Pro-Cys(StBu)-Pro-Ala-Pro-OH (XIII)

1) $(C_4H_9)_3P$
2) $O_2$

H-Leu-[Glu]$_5$-Ala-Tyr-Gly-Trp-Nle-Asp-Phe-Thr-Cys-Pro-Pro-Cys-Pro-Ala-Pro-OH
H-Leu-[Glu]$_5$-Ala-Tyr-Gly-Trp-Nle-Asp-Phe-Thr-Cys-Pro-Pro-Cys-Pro-Ala-Pro-OH (XI)